# EUROPÄISCHE PATENTANMELDUNG

(11) **EP 3 459 636 A1**
(43) Veröffentlichungstag der Anmeldung: **27.03.2019**
(21) Anmeldenummer: 17192547.2
(22) Anmeldetag: 22.09.2017
(51) Int. Cl.: B01L 9/00, B01F 11/00, B01L 3/00

(54) **INKUBATIONSMODUL**

(71) Anmelder: Euroimmun Medizinische Labordiagnostika AG, 23560 Lübeck (DE)
(72) Erfinder: RATEIKE, Martin, 23689 Pansdorf (DE); KAFFKA, Christian, 23942 Dassow (DE); STÖCKER, Winfried, 23627 Groß Grönau (DE)

(57) **Zusammenfassung**

Die vorliegende Erfindung betrifft ein Inkubationsmodul, eine Vorrichtung zur patohistologischen Untersuchung von biologischen Proben und ein Verfahren zum Inkubieren einer vorbestimmten Anzahl von Trägern mit biologischen Proben. Das Inkubationsmodul (1) umfasst ein Array (9) mit einer Vielzahl von Wipptischen (4), wobei jeder Wipptisch (4) aus einer Nulllage heraus um einen vorbestimmten Winkelbereich schwenkbar angeordnet ist und jeder Wipptisch (4) eine Vielzahl von Inkubationsrinnen (2) aufweist, wobei jede Inkubationsrinne (2) eine von den Wänden der Inkubationsrinne (2) gebildete Vertiefung (3) und einen Boden aufweist, wobei jede Inkubationsrinne (2) weiter ein Mittel zum Absaugen von Flüssigkeit (7) an wenigstens einem Längsende der Vertiefung (3) aufweist,
wobei das Mittel zum Absaugen, das Absaugen der Flüssigkeit (7) am Boden gestattet und vorzugsweise eine in einen Auslasskanal (6) mündende Öffnung ist, die sich am Boden der Inkubationsrinne (2) befindet, wobei jede Inkubationsrinne (2) über ihre Querachse schwenkbar angeordnet und mit einem Träger (12) bestückt ist, wobei der Träger (12) und die Inkubationsrinne (2) derart aneinander angepasst sind, dass der Träger (12) in der Vertiefung (3) entlang der Längsachse der Inkubationsrinne (2) hin- und her bewegt werden kann, dadurch, dass die Inkubationsrinne (2) mit einer Drehachse (15) über die Querachse schwenkbar ist, und wobei jede Inkubationsrinne (2) weiter einen Träger mit einer biologischen Probe (14) aufweist, die auf dem Träger vorzugsweise auf der Seite des Trägers angeordnet ist, die dem Boden der Inkubationsrinne (2) zugewandt ist, vorzugsweise auf dem Boden aufliegend.

## Beschreibung

Die vorliegende Erfindung betrifft ein Inkubationsmodul, eine Vorrichtung zur patohistologischen Untersuchung von biologischen Proben und ein Verfahren zum Inkubieren einer vorbestimmten Anzahl von Trägern mit biologischen Proben.

Pathologische und labordiagnostische Untersuchungen stellen eine unverzichtbare Grundlage für die moderne Medizin dar. Mittlerweile steht eine Vielzahl von routinemäßig durchführbaren Tests zur Verfügung, mit Hilfe derer in Abwesenheit des Patienten aus Probenmaterial entscheidende Informationen zum vorliegenden Krankheitsbild, zur Prognose oder zum Erfolg einer Behandlung erhalten werden können.

Dabei können Patientenproben in Form von Gewebeschnitten oder Zellen zunächst angefärbt und die angefärbten Strukturen im Rahmen einer Befunderstellung dann untersucht werden. Insbesondere im Bereich der Histologie bzw. Histopathologie werden mikrometerdünne, gefärbte Gewebsschnitte hergestellt und am Mikroskop beurteilt.

Zum Probengut beim histologischen Arbeiten gehören vor allem Operationspräparate, Probeexzisionen sowie mittels Biopsien entnommenes Gewebe, wobei das vorrangige Ziel bei der Untersuchung derart angefärbter Gewebeschnitte in der sicheren Erkennung und Typisierung von Tumoren liegt. Mit Hilfe derartiger Verfahren können Gewebe histologisch charakterisiert und über die Untersuchungen von Wucherungen und Tumoren Krebs diagnostiziert werden.

Alternativ können Patientenproben auf das Vorhandensein oder die Konzentration bestimmter Moleküle untersucht werden, wenn die erhaltenen Werte anhand von Referenzdaten für die Diagnose nützliche Information darstellen. So kann der Nachweis von spezifischen Autoantikörpern anzeigen, dass der Patient unter einer Autoimmunkrankheit leidet. Beispielhafte Autoimmunkrankheiten umfassen entzündliche Krankheiten wie rheumatoide Krankheiten, Stoffwechselkrankheiten wie Diabetes und neurologische Erkrankungen.

Ein ungelöstes Problem ist die Knappheit und Hochpreisigkeit von Reagenzien und Probenmaterial. Wirtschaftlichkeitserwägungen führen dazu, dass Arbeitsabläufe und Materialverbrauch optimiert werden. Insbesondere geht der Trend in Richtung Miniaturisierung: diagnostische, pathohistologische und analytische Reaktionen werden nicht mehr im Maßstab von Millilitern, sondern von Mikro- oder gar Nanolitern ausgeführt. Das spart Reagenzien, Platz und gestattet, dass eine einmal gewonnene Probe genug Ausgangsmaterial für eine große Anzahl von diagnostischen Untersuchungen liefert. Nicht zuletzt bleibt dem Patienten auch für den Fall, dass ein einzelner diagnostischer Test misslingt, die erneute Entnahme einer Probe erspart.

Ein besonderes Problem bei der Miniaturisierung besteht darin, dass das Verhältnis des durch Kapillarkräfte und Adhäsion an Oberflächen zurückgehaltenen Flüssigkeitsvolumens einerseits und des Gesamtvolumens der für einen Verfahrensschritt eingesetzten Flüssigkeit andererseits besonders hoch ist. Mit anderen Worten haftet dermaßen viel Flüssigkeit an den Oberflächen, dass diese nach einem Verfahrensschritt durch Abgießen oder -pipettieren nicht effizient entfernt werden kann.

Es verbleibt ein relativ großes Volumen der Flüssigkeit, das nachfolgende Verfahrensschritte stört. Beispielsweise verdünnt eine verbliebene Waschlösung ein für eine nachfolgende Reaktion eingebrachtes Reagenz und verringert dadurch die Ausbeute bzw. Empfindlichkeit der nachfolgenden Reaktion.

Des Weiteren ist es problematisch, wenn einzelne Proben individuell behandelt während sollen oder müssen, insbesondere während andere Proben im selben Prozess geschwenkt bzw. gewippt werden. Eine individuelle Ansteuerung und Prozessierung von Proben ist bei pathologischen und labordiagnostischen Untersuchungen nur sehr umständlich oder gar nicht möglich.

US 2010/0124750 A1 beschreibt eine Vorrichtung zur Durchführung von immunologischen, histochemischen und zytochemischen, molekularbiologischen, enzymologischen, klinischchemischen und anderen Analysen, wobei die Vorrichtung einen Objektträger mit einem oder mehreren länglichen Klebeflächen und einen Reagenzhalter mit einem oder mehreren Kanälen aufweist.

WO 2016/169576 A1 beschreibt eine Inkubationsrinne mit einer von den Wänden der Inkubationsrinne gebildeten Vertiefung und einem Boden, wobei die Inkubationsrinne ein Mittel zum Absaugen von Flüssigkeit an wenigstens einem Längsende der Vertiefung aufweist, bevorzugt eine in einen Auslasskanal mündende Öffnung an dem Längsende der Vertiefung, besonders bevorzugt in einer Wand der Inkubationsrinne, wobei Öffnung und Auslasskanal so ausgeführt sind, dass Unterdruck angelegt werden kann, wobei die Inkubationsrinne über ihre Querachse schwenkbar ist, und wobei die Inkubationsrinne mit einem Träger bestückt werden kann.

Der Erfindung liegt die Aufgabe zugrunde, eine Möglichkeit bei pathologisch oder labordiagnostisch zu untersuchenden Proben bereitzustellen, wodurch eine individuelle Behandlung einzelner Proben ermöglicht wird, und wobei eine Zeitersparnis und eine Erhöhung der Flexibilität erzielt werden.

Diese Aufgabe wird durch ein Inkubationsmodul nach Anspruch 1, eine Vorrichtung zur patohistologischen Untersuchung von biologischen Proben nach Anspruch 11 und ein Verfahren zum Inkubieren einer vorbestimmten Anzahl von Trägern nach Anspruch 12 gelöst. Vorteilhafte Weiterbildungen sind in den Unteransprüchen definiert.

Erfindungsgemäß wird die Aufgabe in einem ersten Aspekt gelöst durch ein Inkubationsmodul umfassend ein Array mit einer Vielzahl von Wipptischen, wobei jeder Wipptisch aus einer Nulllage heraus um einen vorbestimmten Winkelbereich schwenkbar angeordnet ist und jeder Wipptisch eine Vielzahl von Inkubationsrinnen aufweist. Jede Inkubationsrinne weist eine von den Wänden der Inkubationsrinne gebildete Vertiefung und einen Boden auf, wobei jede Inkubationsrinne weiter ein Mittel zum Absaugen von Flüssigkeit an wenigstens einem Längsende der Vertiefung aufweist, vorzugsweise eine in einen Auslasskanal mündende Öffnung an dem Längsende der Vertiefung, besonders vorzugsweise in einer Wand der Inkubationsrinne, wobei Öffnung und Auslasskanal so ausgeführt sind, dass Unterdruck angelegt werden kann, wobei das Mittel zum Absaugen, das Absaugen der Flüssigkeit am Boden gestattet und vorzugsweise eine in einen Auslasskanal mündende Öffnung ist, die sich am Boden der Inkubationsrinne befindet. Jede Inkubationsrinne ist über ihre Querachse schwenkbar angeordnet und mit einem Träger bestückt, wobei der Träger und die Inkubationsrinne derart aneinander angepasst sind, dass der Träger in der Vertiefung entlang der Längsachse der Inkubationsrinne hin- und her bewegt werden kann, dadurch, dass die Inkubationsrinne mit einer Drehachse über die Querachse schwenkbar ist, und wobei jede Inkubationsrinne weiter einen Träger mit einer biologischen Probe aufweist, die auf dem Träger vorzugsweise auf der Seite des Trägers angeordnet ist, die dem Boden der Inkubationsrinne zugewandt ist, vorzugsweise auf dem Boden aufliegend.

Somit wird eine individuelle Behandlung einzelner biologischer Proben ermöglicht. Das Array umfasst vorzugsweise m Reihen in einer ersten definierten Richtung und n Reihen in einer zweiten definierten Richtung, die zur ersten definierten Richtung orthogonal ist, wobei m und n natürliche Zahlen sind, besonders vorzugsweise sind m und n gleiche natürliche Zahlen. Die biologische Probe ist vorzugsweise ein Gewebeschnitt, besonders vorzugsweise ein Gewebeschnitt von Humangewebe, ein Zellschnitt oder ein Protein.

In einer bevorzugten Ausführungsform des ersten Aspekts weist jeder Wipptisch eine Koppelmechanikeinheit auf, die ein Auskoppeln eines einzelnen Wipptischs aus dem Array gestattet. Dadurch muss nicht der gesamte Prozess unterbrochen werden, wenn nur ein Wipptisch bzw. eine oder wenige Probe(n) betroffen ist/sind, was zur Erhöhung der Flexibilität und auch zu einer Zeitersparnis führt, insbesondere können Proben zu unterschiedlichen Zeiten in den Gesamtprozess eingebunden bzw. ausgekoppelt werden.

In einer weiteren bevorzugten Ausführungsform weist jeder Wipptisch eine Arretiereinheit auf, die ein Fixieren des einzelnen Wipptischs in dem Array gestattet. Dies ist vorteilhaft bezüglich der Bearbeitung einzelner Proben, denn dies erhöht weiter die Flexibilität.

In einer weiteren bevorzugten Ausführungsform wird ein einzelner Wipptisch unabhängig von den anderen Wipptischen aus dem Array fixiert, so dass eine weitere biologische Probe auf den einzelnen Wipptisch beladen wird und/oder die biologische Probe von dem einzelnen Wipptisch entladen wird. Damit muss nicht der Prozess abgebrochen und von neuem beginnen, sondern der Prozess läuft weiter während eine neue Probe beladen wird und/oder eine alte Probe entladen wird. Dies führt zu einer enormen Zeitersparnis und auch zu einer höheren Flexibilität, da unterschiedliche Proben am gleichen Tag bzw. zur gleichen Zeit bearbeitet werden können.

In weiteren bevorzugten Ausführungsformen sind Koppelmechanikeinheit und Arretiereinheit einstückig ausgebildet. Dies führt zu einer kompakten Bauform und damit zu einer erhöhten Platzeinsparung.

In einer bevorzugten Ausführungsform des ersten Aspekts weist das Inkubationsmodul weiter eine Transporteinheit auf, die bewegbar angeordnet ist und jeden Wipptisch einzeln oder synchron eine Teilmenge der Wipptische aus dem Array greifen und transportieren kann. Somit wird eine individuelle Behandlung einzelner Proben oder einer Teilmenge von Proben, insbesondere während andere Proben geschwenkt werden, ermöglicht. Vorzugsweise ist jeder Wipptisch einzeln oder ist synchron eine Teilmenge der Wipptische aus dem Array in dem vorbestimmten Winkelbereich durch die Transporteinheit befestigbar.

In einer weiteren bevorzugten Ausführungsform des ersten Aspekts weist das Inkubationsmodul weiter eine Pipettiereinheit auf. In anderen bevorzugten Ausführungsformen des ersten Aspekts weist das Inkubationsmodul weiter eine Spüleinheit auf. Vorzugsweise arretiert neben der Transporteinheit auch die Pipettiereinheit die Wipptische aus jeder Wipplage heraus für die weitere Bearbeitung. Vorteilhafterweise arretiert die jeweilige mechanische Komponente, d.h. Transporteinheit, Spüleinheit oder Pipettiereinheit, für die Bearbeitungsdauer den jeweilig zu bearbeitenden Wipptisch eigenständig für den auszuführenden Arbeits- bzw. Bearbeitungsschritt.

In einer bevorzugten Ausführungsform des ersten Aspekts ist jeder Wipptisch einzeln durch ein Kühl- und/oder Heizelement temperierbar. Damit sind unterschiedliche Bearbeitungstemperaturen der Proben auf den einzelnen Wipptischen in dem Array möglich. Dies ist auch bezüglich der Flexibilität vorteilhaft.

In einer weiteren bevorzugten Ausführungsform des ersten Aspekts ist das Array über einen zentralen Antrieb, vorzugsweise über einen Schrittmotor, schwenkbar angeordnet. In anderen bevorzugten Ausführungsformen werden andere Linearmotoren und/oder Rotationsmotoren verwendet.

In einer bevorzugten Ausführungsform des ersten Aspekts umfasst der vorbestimmte Winkelbereich einen Bereich von -35° bis +35°, vorzugsweise einen Bereich von -25° bis +25°, besonders vorzugsweise einen Bereich von -20° bis +20° um die Nulllage herum. Der Winkelbereich von -25° bis +25° begünstigt vorteilhafterweise eine Inkubation. Die Winkelbereiche von -25° bis +25° und von -35° bis +35° begünstigen vorteilhafterweise den Flüssigkeitsaustausch in der Inkubationsrinne unter Ausnutzung der Schwerkraft. Des Weiteren wird ein Absaugen begünstigt, ohne dass Inkubationsbereiche in der Inkubationsrinne in einer maximalen Wipplage trocken liegen. In anderen bevorzugten Ausführungsformen umfasst der vorbestimmte Winkelbereich einen Bereich von -90° bis +90° um die Nulllage herum. Der vorbestimmte Winkelbereich ermöglicht das Einstellen des Wippwinkels, vorzugsweise eine Anpassung des Wippwinkels an die Viskosität der Flüssigkeit, um einerseits eine optimale Flüssigkeitsverteilung während der Inkubation zu erreichen und um andererseits zu verhindern, dass Inkubationsbereiche in der Inkubationsrinne in einer maximalen Wipplage trocken liegen. Durch das Einstellen des Wippwinkels wird vorzugsweise ein Auslaufen der Flüssigkeit bzw. ein Überkippen der Inkubationsrinne vermieden.

In einer weiteren bevorzugten Ausführungsform weist die Inkubationsrinne eine in den Auslasskanal mündende Öffnung entlang der Längsachse der Inkubationsrinne am einen Längsende und eine in einen Einlasskanal mündende Öffnung am anderen Längsende der Vertiefung auf. Somit kann ein reproduzierbarer Prozess erreicht werden.

In einer weiteren bevorzugten Ausführungsform kann der Träger beim Bewegen bis an ein Längsende der Vertiefung bewegt werden und dabei lückenlos an das Längsende der Vertiefung anschließen. Damit wird die Reproduzierbarkeit des Prozesses noch weiter erhöht.

Erfindungsgemäß wird die Aufgabe in einem zweiten Aspekt gelöst durch eine Vorrichtung zur patohistologischen Untersuchung von biologischen Proben, umfassend eine Halterung für wenigstens ein Inkubationsmodul nach dem ersten Aspekt.

Erfindungsgemäß wird die Aufgabe in einem dritten Aspekt gelöst durch ein Verfahren zum Inkubieren einer vorbestimmten Anzahl von Trägern, umfassend die Schritte: a) Inkubation eines Trägers in einer von den Wänden der Inkubationsrinne gebildeten Vertiefung und einem Boden in Gegenwart einer Flüssigkeit, und b) Absaugen der Flüssigkeit an einem Längsende der Vertiefung, vorzugsweise über eine Öffnung, die in einen unter Unterdruck stehenden Auslasskanal mündet, wobei sich die Öffnung am Längsende der Vertiefung befindet, vorzugsweise am Boden der Inkubationsrinne, und wobei die Schritte a) und b) solange wiederholt werden bis eine vorbestimmte Anzahl von Trägern in einer Vielzahl von Inkubationsrinnen, enthaltend in einem Inkubationsmodul gemäß dem ersten Aspekt oder in einer Vorrichtung gemäß dem zweiten Aspekt, mit wenigstens einer biologischen Probe (14) beladen wird.

In einer bevorzugten Ausführungsform des dritten Aspekts wird wenigstens einer der Schritte a) und b), vorzugsweise Schritt b) unter Schwenken in einem vorbestimmten Winkelbereich durchgeführt.

In einer weiteren bevorzugten Ausführungsform wird Schritt b) in dem Moment ausgeführt, in dem eine Inkubationsrinne derart geschwenkt wird, dass eine darin befindliche Flüssigkeit sich an dem Längsende der Inkubationsrinne ansammelt, an dem die Flüssigkeit abgesaugt wird, und in dem vorzugsweise der Träger sich bis an dieses Längsende der Vertiefung bewegt hat und besonders vorzugsweise dabei lückenlos an das Längsende der Vertiefung anschließt.

Die vorliegende Erfindung betrifft also ein Inkubationsmodul, eine Vorrichtung zur patohistologischen Untersuchung von biologischen Proben und ein Verfahren zum Inkubieren einer vorbestimmten Anzahl von Trägern mit biologischen Proben. Das Inkubationsmodul umfasst eine Vielzahl von Inkubationsrinnen, an die zur Entfernung von Flüssigkeit Unterdruck angelegt wird. Jede Inkubationsrinne ist vorzugsweise so ausgestaltet, dass auf diese Weise auch Flüssigkeitsreste entfernt werden können, die durch ein ausschließlich schwerkraftgetriebenes Abgießen nicht entfernt werden würden. Die Flüssigkeit wird daher effizienter entfernt als mit herkömmlichen Inkubationsmodulen und Verfahren.

Somit wird eine Möglichkeit bereitgestellt, eine individuelle Behandlung einzelner biologischer Proben zu ermöglichen, wobei eine erhebliche Zeitersparnis und eine Erhöhung der Flexibilität erreicht werden. Vorteilhafterweise ist eine individuelle Behandlung einzelner Proben, insbesondere während andere geschwenkt werden, möglich. Es ist also vorteilhaft, dass eine Vielzahl solcher Inkubationsrinnen bzw. solcher Wipptische gleichzeitig benutzt wird und insbesondere, dass diese individuell angesteuert und prozessiert werden können. Weiterhin ist vorteilhaft, dass jeder Wipptisch separat justierbar, pipettierbar, waschbar, befüllbar, entladbar, transportierbar, schwenkbar, vorzugsweise wippbar, und/oder temperierbar ist.

Im Folgenden wird die Erfindung unter Bezugnahme auf die Figuren anhand von Ausführungsbeispielen erläutert. Die beschriebenen Ausführungsbeispiele sind in jeder Hinsicht lediglich beispielhaft und nicht als einschränkend zu verstehen, und verschiedene Kombinationen der angeführten Merkmale sind vom Umfang der Erfindung umfasst.
**Fig. 1** zeigt ein Inkubationsmodul mit fünfundzwanzig Wipptischen und fünfundzwanzig Inkubationsrinnen gemäß einem ersten bevorzugten Ausführungsbeispiel der Erfindung.
**Fig. 2** zeigt ein Array mit Wipptischen um 20°nach rechts aus der Nulllage ausgeschwenkt gemäß einem zweiten bevorzugten Ausführungsbeispiel der Erfindung.
**Fig. 3** zeigt eine Transporteinheit, die sich senkrecht auf den zu bearbeitenden Wipptisch absenkt gemäß einem dritten bevorzugten Ausführungsbeispiel der Erfindung.
**Fig. 4** zeigt die Transporteinheit gemäß dem dritten bevorzugten Ausführungsbeispiel der Erfindung, die den zu bearbeitenden Wipptisch zum Fixieren eingefangen hat.
**Fig. 5A** zeigt eine Inkubationsrinne mit einer Vertiefung in einer Draufsicht gemäß einem vierten bevorzugten Ausführungsbeispiel der Erfindung.
**Fig. 5B** zeigt eine Inkubationsrinne im Querschnitt an der in **Fig. 5A** mit dem schwarzen Strich markierten Stelle gemäß dem vierten bevorzugten Ausführungsbeispiel der Erfindung.
**Fig. 6A** zeigt eine mit einer Flüssigkeit gefüllte Inkubationsrinne mit einer Vertiefung, die mit einem Träger mit einer biologischen Probe bestückt ist gemäß einem fünften bevorzugten Ausführungsbeispiel der Erfindung.
**Fig. 6B** zeigt die gleiche Vorrichtung nach dem Schwenken über die Querachse gemäß dem fünften bevorzugten Ausführungsbeispiel der Erfindung.
**Fig. 6C** zeigt, dass Träger, Boden und Wände der Inkubationsrinne in diesem fünften bevorzugten Ausführungsbeispiel der Erfindung einen Kanal bilden, durch den die Flüssigkeit durch den Auslasskanal besonders effizient abgesaugt werden kann, wenn Unterdruck angelegt wird.

In der vorliegenden Beschreibung versteht es sich, wenn ein Element "auf" einem anderen Element beschrieben wird, dass das Element direkt auf dem anderen Element angeordnet sein kann oder dass dazwischen liegende Elemente vorhanden sind. Im Gegensatz dazu versteht es sich, dass wenn ein Element als "direkt auf" einem anderen Element beschrieben wird, dann gibt es keine dazwischen liegenden Elemente. Es versteht sich weiterhin, wenn ein Element "unter" einem anderen Element beschreiben wird, dass das Element direkt unter dem anderen Element angeordnet sein kann oder dass dazwischen liegende Elemente vorhanden sind. Im Gegensatz dazu versteht es sich, dass wenn ein Element als "direkt unter" einem anderen Element beschreiben wird, dann gibt es keine dazwischen liegenden Elemente.

Weiterhin können relative Begriffe wie "unten" oder "oben" verwendet werden, um die Beziehung eines Elements zu anderen Elementen zu beschreiben, wie in den Figuren dargestellt. Es versteht sich, dass relative Begriffe dazu dienen, unterschiedliche Orientierungen der erfindungsgemäßen Vorrichtungen zusätzlich zu der in den Figuren dargestellten Orientierung zu umfassen. Wenn zum Beispiel eine Vorrichtung in einer der Figuren umgedreht wird, würden Elemente, die als auf der "unteren" Seite anderer Elemente beschrieben werden, dann auf "oberen" Seiten der anderen Elemente ausgerichtet sein. Die beispielhaften Begriffe "unten" oder "darunter" können daher sowohl eine Orientierung von oben als auch unten umfassen. Sofern ein strukturelles Merkmal als "unter" oder "über" dem Boden, seiner Ebene oder einer zu dieser Ebene parallelen Ebene lokalisiert angegeben ist, ist damit die Lage relativ zu dieser Ebene vertikal gemeint.

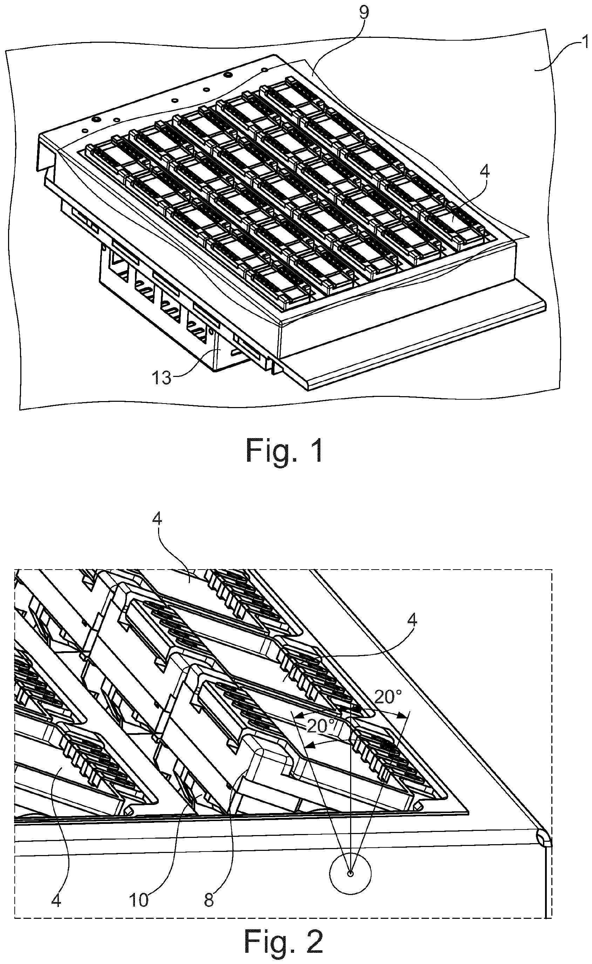

**Fig. 1** zeigt ein Inkubationsmodul 1 mit fünfundzwanzig Wipptischen und fünfundzwanzig Inkubationsrinnen gemäß einem ersten bevorzugten Ausführungsbeispiel der Erfindung, wobei sich die Wipptische 4 in der Nulllage befinden. Das Inkubationsmodul 1 verfügt über fünfundzwanzig Wipptische, die aus der Nulllage heraus um jeweils 20° nach links und rechts, d. h. um -20° bzw. um +20°, schwenkbar sind. Das Array 9 mit fünfundzwanzig Wipptischen 4 ist über einen zentralen Antrieb schwenkbar, in diesem ersten bevorzugten Ausführungsbeispiel ein Schrittmotor 13. Das Array 9 hat in diesem ersten bevorzugten Ausführungsbeispiel fünf Reihen in einer ersten definierten Richtung einer Ebene und fünf Reihen in einer zweiten definierten Richtung der Ebene, die zur ersten definierten Richtung orthogonal ist. Mit anderen Worten, das Array 9 hat 5x5 Reihen, besteht also insgesamt aus fünfundzwanzig Wipptischen 4.

**Fig. 2** zeigt ein Ausschnitt aus einem Array 9 mit Wipptischen 4 um 20°nach rechts aus der Nulllage ausgeschwenkt gemäß einem zweiten bevorzugten Ausführungsbeispiel der Erfindung. Jeder Wipptisch 4 verfügt über eine Koppelmechanikeinheit 8 für die Wipp-Bewegungsübertragung. Die Koppelmechanikeinheit 8 ermöglicht das Auskoppeln eines einzelnen Wipptischs 4 aus der zentral mit dem Antrieb erzeugten Wippbewegung. Neben der Koppelmechanikeinheit 8 befindet sich eine Arretiereinheit 10 zur Fixierung des einzelnen Wipptischs 4. In diesem zweiten bevorzugten Ausführungsbeispiel hat jeder Wipptisch eine eigene Arretiereinheit 10 und der Wipptisch 4 wird fixiert, d.h. die Wippbewegung wird unterbrochen, und eine Probe 14 beladen.

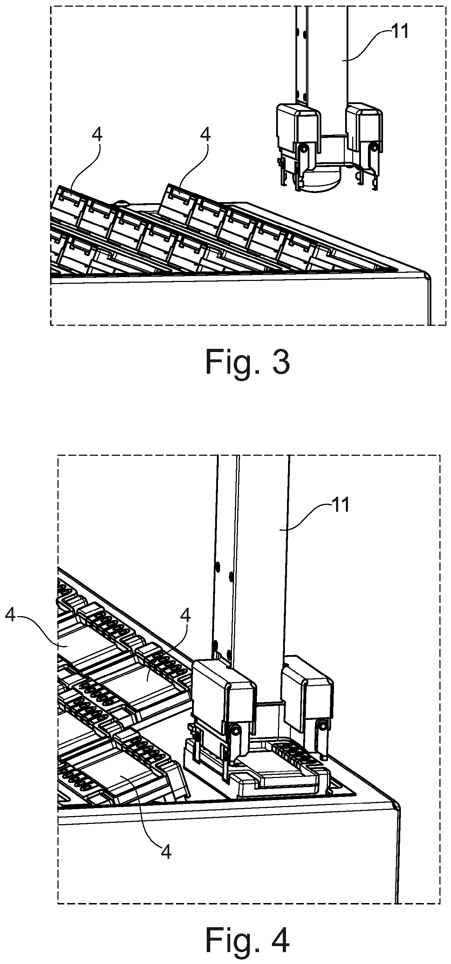

**Fig. 3** zeigt eine Transporteinheit 11, die sich senkrecht auf den zu bearbeitenden Wipptisch 4 absenkt gemäß einem dritten bevorzugten Ausführungsbeispiel der Erfindung. Die Wipptische 4 werden jeweils über die Transporteinheit 11 aus der Wippbewegung eingefangen und in die Bearbeitungsposition gebracht, um diese dann über die Tischarretierung zu fixieren. Die Fixierung kann je nach Bedarf ohne die Transporteinheit 11 beliebig lang über die Tischarretierung beibehalten werden.

**Fig. 4** zeigt die Transporteinheit 11 gemäß dem dritten bevorzugten Ausführungsbeispiel der Erfindung, die den zu bearbeitenden Wipptisch 4 zum Fixieren eingefangen hat, wobei der Wipptisch 4 aus dem Array 9 auskoppelt ist.

In anderen bevorzugten Ausführungsbeispielen wird durch die jeweilige Koppelmechanikeinheit 8 ermöglicht, dass nur ein zentraler Antrieb benötigt wird, um ein Array 9 von Wipptischen 4 synchron wippen zu lassen. Des Weiteren wird nur der Wipptisch 4 fixiert, der auch bearbeitet wird. Alle anderen Wipptische 4 wippen für die Inkubation weiter. Das Auskoppeln erfolgt direkt über die Transporteinheit 11, so dass diese den jeweiligen Wipptisch 4 aus jeder beliebigen Wipplage allein über die Absenkbewegung in die benötigte Bearbeitungsposition ausrichten können.

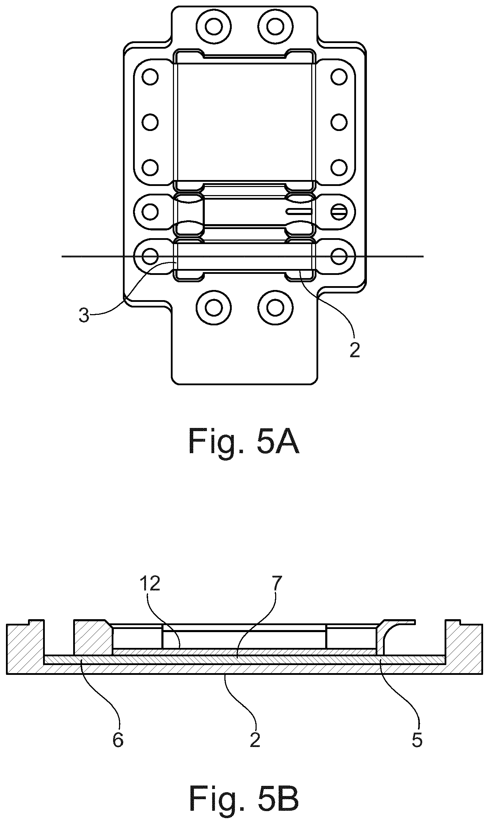

**Fig. 5A** zeigt eine Inkubationsrinne 2 mit einer Vertiefung 3 in einer Draufsicht gemäß einem vierten bevorzugten Ausführungsbeispiel der Erfindung. Der schwarze Strich markiert die Stelle, an der der in **Fig. 5B** dargestellte Querschnitt liegt.

**Fig. 5B** zeigt eine Inkubationsrinne 2 im Querschnitt an der in **Fig. 5A** mit dem schwarzen Strich markierten Stelle gemäß dem vierten bevorzugten Ausführungsbeispiel der Erfindung. Ein Träger 12 mit einer biologischen Probe ist in die Flüssigkeit 7 eingetaucht. Außerdem sind Einlasskanal 5 und Auslasskanal 6 zu erkennen.

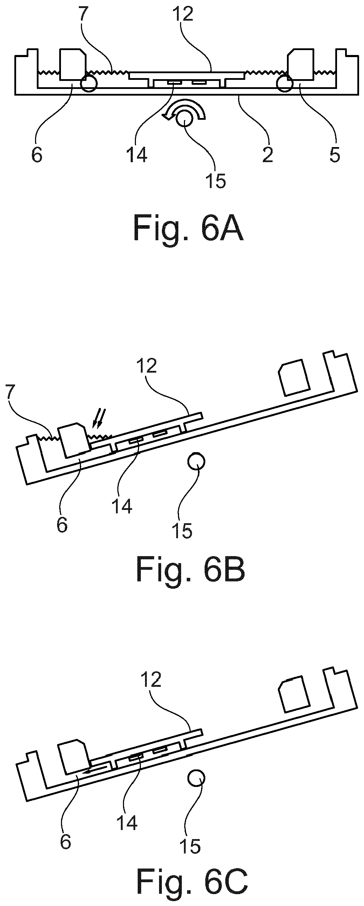

**Fig. 6A** zeigt eine mit einer Flüssigkeit 7 gefüllte Inkubationsrinne 2 mit einer Vertiefung 3, die mit einem Träger 12 mit einer biologischen Probe 14 bestückt ist gemäß einem fünften bevorzugten Ausführungsbeispiel der Erfindung. Die Probe 14 befindet sich auf dem Träger 12 auf der dem Boden der Inkubationsrinne 2 zugewandten Seite. Der Träger 12 kann entlang der Längsachse in der Inkubationsrinne 2 hin- und her bewegt werden, insbesondere dadurch, dass die Inkubationsrinne 2 mit einer Drehachse 15 über die Querachse (nicht dargestellt, parallel zur Drehachse) geschwenkt wird. Der Träger 12 steht mit zwei Füßen auf dem Boden, die so ausgeführt sind, dass Flüssigkeit 7 entlang der Längsachse an ihnen vorbeiströmen kann.

**Fig. 6B** zeigt die gleiche Anordnung nach dem Schwenken über die Querachse gemäß dem fünften bevorzugten Ausführungsbeispiel der Erfindung. Der Träger 12 mit der biologischen Probe 14 bewegt sich mit der Flüssigkeit 7 zusammen der Schwerkraft folgend, bis zum Längsende der Vertiefung 3 der Inkubationsrinne 2, wobei der Träger 12 lückenlos an das Längsende der Vertiefung 3 anschließt (mit einem Doppelpfeil markiert). Die Flüssigkeit 7 sammelt sich am nach unten geschwenkten Längsende der Vertiefung 3 bzw. der Inkubationsrinne 2 an.

**Fig. 6C** zeigt, dass Träger 12, Boden und Wände der Inkubationsrinne 2 in diesem fünften bevorzugten Ausführungsbeispiel der Erfindung einen Kanal bilden, durch den die Flüssigkeit 7 durch den Auslasskanal 6 besonders effizient abgesaugt werden kann, wenn Unterdruck angelegt wird. Der Strom der Flüssigkeit 7 durch den Kanal ist mit einem einfachen Pfeil markiert. Es verbleiben am Längsende keine Flüssigkeitsreste.

In einem sechsten bevorzugten Ausführungsbeispiel ist jede Inkubationsrinne 2 flüssigkeitsdicht. Sie kann mit einem Deckel, einer Folie oder einer ähnlichen Abdeckung versehen sein. Die Inkubationsrinne 2 kann in Form eines einlegbaren Einsatzes in ein daran angepasstes Tablett einsetzbar sein, das eine Vielzahl von Inkubationsrinnen, z.B. wenigstens 2, 3, 4, 5, 6, 10, 12, 15, 20, 30 oder mehr Inkubationsrinnen, enthalten kann. In anderen bevorzugten Ausführungsbeispielen kann das Tablett mehrere Inkubationsrinnen unterschiedlicher Größe aufnehmen. Der Boden der Inkubationsrinne 2 ist flach und bildet eine Ebene. Nichtsdestotrotz muss es sich keinesfalls um eine Ebene ohne jegliche Unregelmäßigkeiten handeln, vielmehr kann in anderen bevorzugten Ausführungsbeispielen der Boden mit Rillen, Streifen oder Aufrauhungen versehen sein.

Die Inkubationsrinne 2 ist in dem sechsten bevorzugten Ausführungsbeispiel mit einem Träger 12 bestückt. Dabei handelt es sich vorzugsweise um einen flachen, an die Breite der Inkubationsrinne 2 angepassten Träger 12, vorzugsweise um einen Glasträger. Der Träger 12 ist also transparent und kann direkt mit einer Probe 14 beschichtet sein, es ist jedoch auch möglich, die Probe 14 ihrerseits auf einen weiteren Objektträger, beispielsweise eine Folie oder eine dünne Glas- oder Kunststoffscheibe aufzutragen, die auf den transparenten Objektträger geklebt oder auf andere Weise darauf befestigt wird. Vorzugsweise ist die Probe 14 an der Unterseite des Trägers 12 angeordnet, d. h. der Seite, die dem Boden der Inkubationsrinne 2 zugewandt ist.

In diesem sechsten bevorzugten Ausführungsbeispiel sind Inkubationsrinne 2 und Träger 12 derart aneinander angepasst, dass die Inkubationsrinne 2 mit dem Träger 12 bestückt wird. Vorzugsweise sind Inkubationsrinne 2 und Träger 12 länglich, wobei die Länge des Trägers 12 die Länge der Vertiefung 3 unterschreitet, so dass der in die Vertiefung 3 eingelegte Träger 12 nicht beide Längsenden der Vertiefung 3 berührt. Vorzugsweise beträgt die Länge des Trägers 12 50 bis 99 Prozent, besonders vorzugsweise 50 bis 95 Prozent, noch besonders vorzugsweise 60 bis 90 Prozent der Länge der Vertiefung 3. Die Breite des Trägers 12 ist vorzugsweise so gewählt, dass sich der in die Vertiefung 3 eingebrachte Träger 12 nicht auf dem Boden der Inkubationsrinne 2 drehen kann.

Der in die Vertiefung 3 eingebrachte Träger 12 ist vorzugsweise unbefestigt und kann sich beim Schwenken der Inkubationsrinne 2 mit Träger 12 und Flüssigkeit 7 darin im Rahmen der geometrischen Begrenzungen durch die Wände der Vertiefung 3 bewegen, vorzugsweise entlang der Längsachse. Vorzugsweise sind die Form von Inkubationsrinne 2 und Träger 12 derart aneinander angepasst, dass ein Ende des Trägers 12 lückenlos an das Längsende der Vertiefung 3 anschließt, wenn die Inkubationsrinne 2 derart geschwenkt wird, dass sich der Träger 12 bis zum Ende der Vertiefung 3 bewegt und auf dem Längsende der Vertiefung 3 aufsetzt. Die lückenlose Abschließung muss sich nicht über die gesamte Breite des Trägers 12 erstrecken, reicht aber aus, um unter dem Träger 12 befindliche bzw. darunter strömende Flüssigkeit 7 wenigstens teilweise in Richtung einer Öffnung der Inkubationsrinne 2 zu kanalisieren.

In anderen bevorzugten Ausführungsbeispielen handelt es sich bei dem Träger 12 um einen Teststreifen und/oder um eine geeignete Membran, beispielsweise eine Nitrozellulosemembran, beschichtet mit Proben oder diagnostischen Reagenzien.

In wieder anderen bevorzugten Ausführungsbeispielen liegt der Träger 12 auf dem Boden der Vertiefung 3 derart auf, dass er nach Einfüllen einer Flüssigkeit 7 mit derselben wenigstens teilweise unterschichtet ist, so dass eine Probe 14 auf dem Träger 12 mit der Flüssigkeit 7 in Kontakt steht. Der Träger 12 liegt direkt auf dem Boden auf, oder die Inkubationsrinne 2 und/oder der Träger 12 weist ein Mittel zum Fixieren eines Abstandes, vorzugsweise des Mindestabstandes, zwischen Unterseite des Trägers 12 und Boden der Vertiefung 3 auf. Insbesondere kann auf der Unterseite des Trägers 12 ein oder mehr als ein Fuß angebracht sein, beispielsweise ein Sockel oder eine oder mehr als eine Schiene, optional in Form eines parallel angeordneten Paares, beispielsweise parallel zur Längsrichtung des Trägers 12 an dessen dem Boden zugewandten Seite angebracht, aus dem gleichen Material wie der Träger 12.

In wieder anderen bevorzugten Ausführungsbeispielen liegt auf dem Boden der Inkubationsrinne 2 oder an den Wänden ein Rahmen auf oder ist angebracht, auf dem der Träger 12 in einem zum Boden der Vertiefung 3 fixierten Abstand ruht. Auch auf andere Weise kann der notwendige lokale Flüssigkeitsaustausch gewährleistet werden, z.B. dadurch, dass die Inkubationsrinne 2 bzw. ein Wipptisch 4 oder das Array 9 regelmäßig bewegt wird und der Träger 12 wenigstens kurzzeitig verrückt wird oder ohne Umdrehung aufschwimmt.

Das Mittel zum Fixieren des Abstandes bewirkt, besonders wenn die Probe 14 auf der Unterseite des Trägers 12 angeordnet ist, dass die Probe 14 selbst dann mit Flüssigkeit 7 in Kontakt steht, wenn der Träger 12 nicht vollständig bedeckt ist. Beim Absaugen wird auch Flüssigkeit 7 entfernt, die auf Grund von Wechselwirkung mit Oberflächen an dem Träger 12 oder der Inkubationsrinne 2 haftet und sich durch Abgießen nicht entfernen lassen würde.

In einem weiteren bevorzugten Ausführungsbeispiel ist oder wird die Probe diagnostisch oder analytisch prozessiert. Das bedeutet vorzugsweise, dass die Probe derart mit diagnostischen Verfahren behandelt wurde, dass ihr Zustand, optional nach weiteren Prozessierungsschritten, eine Analyse und einen Beitrag zu einer Diagnose gestattet. Es kommen beispielsweise labormedizinische, zytologische, morphologisch-mikroskopische, biochemische, (immun)chemische, besonders immunhistochemische oder enzymhistochemische, molekularbiologische, histologische, serologische, pathologische, chemische oder physikalische Analyseverfahren in Frage.

In einem weiteren bevorzugten Ausführungsbeispiel ist die Probe eine biologische Probe, die paraffiniert und/oder in Formalinlösung fixiert ist. Es wird ein Gewebeschnitt mit Farbstoffen oder markierten Antikörpern, vorzugsweise fluoreszenzmarkierte Antikörper, angefärbt und mit Hinblick auf die Morphologie der darin enthaltenen Zellen lichtmikroskopisch untersucht. Beispielhafte molekularbiologische Verfahren umfassen in situ-Hybridisierungen wie die Fluoreszenz-in situ-Hybridisierung. Beispielhafte pathologische Verfahren umfassen Färbungen wie die ATPase-, NADH-, Hämatoxylin-/Eosin-, Gomori-Trichrom-, PAS-, und Ölrot-Färbung an Muskelbiopsien. Geeignete Reagenzien, Software und Geräte zum Prozessieren von Proben sind im Handel erhältlich, beispielsweise von der Firma EUROIMMUN AG Medizinische Labordiagnostika, Lübeck.

In anderen bevorzugten Ausführungsbeispielen umfasst die biologische Probe 14 eine aus einer Gruppe ausgewählte Probe umfassend Gewebe, vorzugsweise Gewebeschnitte oder Gewebebiopsien, z. B. Schnellschnitte, biologische Zellen wie eukaryontische oder prokaryontische Zellen oder Produkte daraus, Viren, aufgereinigte, isolierte oder künstlich hergestellte Moleküle wie Nukleinsäuren, Polypeptide, Lipide oder Kohlenhydrate. Vorzugsweise ist eine biologische Probe 14 menschlichen oder tierischen Ursprungs.

In einem bevorzugten Ausführungsbeispiel der Erfindung weist die Inkubationsrinne 2 einen Auslasskanal 6 mit einer Auslasskanalöffnung zur Entfernung von Flüssigkeit 7 aus der Inkubationsrinne 2 über Absaugen auf, der so ausgestaltet ist, dass der Träger 12 beim Absaugen nicht entfernt oder beschädigt wird. In einem weiteren bevorzugten Ausführungsbeispiel handelt es sich um einen Schlauch, dessen Ende in die Flüssigkeit 7 eingeführt wird. Vorzugsweise handelt es sich um einen in die Inkubationsrinne 2 integrierten Kanal. An den Auslasskanal 6 kann zum Absaugen Druck angelegt werden.

Die Breite des Auslasskanals 6 beträgt vorzugsweise 50 % bis 100 %, besonders vorzugsweise 60 % bis 95 %, noch besonders vorzugsweise 75 % bis 95 % der Breite der Vertiefung 3 der Inkubationsrinne 2 oder des Trägers 12, vorzugsweise des Trägers 12.

Die Öffnung des Auslasskanals 6 ist ausgehend von der Ebene des Bodens vertikal so angeordnet, dass wenigstens ein Teil von ihr, vorzugsweise die gesamte Öffnung unterhalb der Oberfläche einer Flüssigkeit 7 liegt, mit der die Inkubationsrinne 2 gefüllt ist, vorzugsweise soweit gefüllt ist, dass der Träger 12 gerade vollständig mit Flüssigkeit 7 bedeckt ist. Vorzugsweise ist die Öffnung am Boden angeordnet. Das ist der Fall, wenn in der Inkubationsrinne 2 befindliche Flüssigkeit 7, von an Oberflächen anhaftenden Resten abgesehen, vollständig ablaufen kann, wenn die Inkubationsrinne 2 derart geneigt ist, dass die Flüssigkeit 7 in Richtung der Öffnung läuft.

In einem weiteren bevorzugten Ausführungsbeispiel steht der Auslasskanal 6 unter Unterdruck, der vorzugsweise wenigstens derart bemessen ist, dass Flüssigkeit 7 in der Vertiefung 3 abgesaugt wird. Die Förderleistung, mit der die Flüssigkeit abgesaugt wird, kann 0,1 bis 10 1/min, vorzugsweise 0,2 bis 5 l/min, besonders vorzugsweise 0,3 bis 3 l/min betragen. Es können übliche Vorrichtungen zum Absaugen verwendet werden, beispielsweise Membran-, Zahnrad-, Kolben- oder Peristaltikpumpen. Das Absaugen der Flüssigkeit 7 kann kontinuierlich oder diskontinuierlich erfolgen. In diesem bevorzugten Ausführungsbeispiel erfolgt das Absaugen kontinuierlich, d. h. es wird nicht der überwiegende Teil der Flüssigkeit 7 in einem Zug, beispielsweise am Ende des Inkubationsvorganges, abgesaugt, sondern in mehreren Schritten. Vorzugsweise wird bei einem Verfahrensschritt, der das Inkubieren des Trägers 12 in einer Flüssigkeit 7 umfasst, zu wenigstens einem Zeitpunkt, vorzugsweise wenigstens 10, 20, 30, 60, 120, 300, 600 Sekunden, 10, 15, 20 oder 30 Minuten lang gleichzeitig Flüssigkeit 7 an einem Ende der Inkubationsrinne 2 eingelassen und am anderen Ende der Inkubationsrinne 2 abgesaugt. Auf diese Weise kommt der Träger 12 stets nur mit unverbrauchter, frischer Flüssigkeit 7 in Kontakt. Dies beschleunigt die Prozessierung des Trägers 12 bzw. der biologischen Probe 14. In anderen bevorzugten Ausführungsbeispielen wird der Träger 12 nach dem Einlassen der Flüssigkeit 7 zunächst darin inkubiert, vorzugsweise wenigstens 10, 20, 30, 60, 120, 300 Sekunden, 10, 15 oder 30 Minuten lang, bevor die Flüssigkeit 7 abgesaugt wird.

Die Inkubationsrinne 2 kann mit einer Flüssigkeit 7 gefüllt sein, vorzugsweise einer wässrigen Flüssigkeit, besonders vorzugsweise einem Waschpuffer oder Reagenz zum Prozessieren einer Probe 14 auf dem Träger 12. Das Volumen der Flüssigkeit 7 wird so bemessen, dass die biologische Probe 14 damit in ausreichendem Kontakt steht. In einem bevorzugten Ausführungsbeispiel ist das Volumen der Flüssigkeit 7 in der Inkubationsrinne 2 so bemessen, dass sie Träger 12 und Probe 14 darauf in horizontaler Lage vollständig bedeckt. In anderen bevorzugten Ausführungsbeispielen ist es so bemessen, dass die Flüssigkeit 7 eine Probe 14 vollständig bedeckt, die an der Seite des Trägers 12 angeordnet ist, welche dem Boden zugewandt ist. Alternativ ist es so bemessen, dass es die Probe 14 nicht andauernd, aber regelmäßig vollständig benetzt, wenn die Inkubationsrinne 2 beim Inkubieren geschwenkt bzw. gewippt wird. Während bei leicht verfügbaren Lösungen wie unspezifischen Waschpuffern, beispielsweise PBS, oder Lösungen zum Entwickeln eines Signals ein Überschuss eingesetzt werden kann, beschränkt sich der Anwender bei anderen Lösungen wie schwierig zu gewinnenden, nur in geringen Volumina verfügbaren Reagenzien, beispielsweise Antikörpern, insbesondere primären Antikörpern, auf das absolut notwendige Mindestvolumen.

Optional weist die Inkubationsrinne 2 einen Einlasskanal 5 auf. Dabei handelt es sich um ein abgeschlossenes Mittel zum Zufluss von Flüssigkeit 7, das nicht druckabschließend ausgeführt sein muss. Der Zufluss kann direkt in die Inkubationsrinne 2 erfolgen.

Das Inkubationsmodul 1 bzw. die Vielzahl von Inkubationsrinnen 2 oder die Vielzahl der Wipptische 4 kann vorzugsweise derart bewegt werden, dass die Flüssigkeit 7 durchmischt und ihre Exposition gegenüber dem jeweiligen Träger 12 gefördert wird, beispielsweise durch Schwenken bzw. Wippen, Vibrieren, Schütteln oder dergleichen. In bevorzugten Ausführungsbeispielen ist die Inkubationsrinne 2 bzw. das Array 9 über ihre Querachse schwenkbar, so dass sich die Flüssigkeit 7 in Richtung des tiefer liegenden Längsendes der Inkubationsrinne 2 verschiebt. Im geschwenkten Zustand, besonders wenn die Auslassöffnung sich am Längsende befindet und die Auslasskanalöffnung sich an deren Boden befindet, ist das Entnehmen der Flüssigkeit 7 besonders einfach und effizient. Beim Schwenken bildet die Inkubationsrinne 2 mit der Grundfläche vorzugsweise einen Winkel von 1 bis 45°, besonders vorzugsweise 2,5° bis 30°, noch besonders vorzugsweise 7,5° bis 25° aus.

## Patentansprüche

1. Inkubationsmodul (1) umfassend:
ein Array (9) mit einer Vielzahl von Wipptischen (4), wobei jeder Wipptisch (4) aus einer Nulllage heraus um einen vorbestimmten Winkelbereich schwenkbar angeordnet ist und jeder Wipptisch (4) eine Vielzahl von Inkubationsrinnen (2) aufweist,
wobei jede Inkubationsrinne (2) eine von den Wänden der Inkubationsrinne (2) gebildete Vertiefung (3) und einen Boden aufweist,
wobei jede Inkubationsrinne (2) weiter ein Mittel zum Absaugen von Flüssigkeit
(7) an wenigstens einem Längsende der Vertiefung (3) aufweist, vorzugsweise eine in einen Auslasskanal (6) mündende Öffnung an dem Längsende der Vertiefung (3), besonders vorzugsweise in einer Wand der Inkubationsrinne (2), wobei Öffnung und Auslasskanal (6) so ausgeführt sind, dass Unterdruck angelegt werden kann,
wobei das Mittel zum Absaugen, das Absaugen der Flüssigkeit (7) am Boden
gestattet und vorzugsweise eine in einen Auslasskanal (6) mündende Öffnung ist, die sich am Boden der Inkubationsrinne (2) befindet,
wobei jede Inkubationsrinne (2) über ihre Querachse schwenkbar angeordnet und mit einem Träger (12) bestückt ist,
wobei der Träger (12) und die Inkubationsrinne (2) derart aneinander angepasst
sind, dass der Träger (12) in der Vertiefung (3) entlang der Längsachse der Inkubationsrinne (2) hin- und her bewegt werden kann, dadurch, dass die Inkubationsrinne (2) mit einer Drehachse (15) über die Querachse schwenkbar ist, und
wobei jede Inkubationsrinne (2) weiter einen Träger mit einer biologischen
Probe (14) aufweist, die auf dem Träger vorzugsweise auf der Seite des Trägers angeordnet ist, die dem Boden der Inkubationsrinne (2) zugewandt ist, vorzugsweise auf dem Boden aufliegend.

2. Inkubationsmodul nach Anspruch 1, wobei jeder Wipptisch (4) eine Koppelmechanikeinheit (8) aufweist, die ein Auskoppeln eines einzelnen Wipptischs (4) aus dem Array (9) gestattet.

3. Inkubationsmodul nach einem der vorhergehenden Ansprüche, wobei jeder Wipptisch (4) eine Arretiereinheit (10) aufweist, die ein Fixieren des einzelnen Wipptischs (4) in dem Array (9) gestattet.

4. Inkubationsmodul nach einem der einem der vorhergehenden Ansprüche, wobei das Inkubationsmodul (1) weiter eine Transporteinheit (11) aufweist, die bewegbar angeordnet ist und jeden Wipptisch (4) einzeln oder synchron eine Teilmenge der Wipptische (4) aus dem Array (9) greifen und transportieren kann.

5. Inkubationsmodul nach Anspruch 4, wobei jeder Wipptisch (4) einzeln oder synchron eine Teilmenge der Wipptische (4) aus dem Array (9) in dem vorbestimmten Winkelbereich durch die Transporteinheit (11) befestigbar ist.

6. Inkubationsmodul nach einem der vorhergehenden Ansprüche, wobei jeder Wipptisch (4) einzeln durch ein Kühl- und/oder Heizelement temperierbar ist.

7. Inkubationsmodul nach einem der vorhergehenden Ansprüche, wobei das Array (9) über einen zentralen Antrieb, vorzugsweise ein Schrittmotor (13), schwenkbar angeordnet ist.

8. Inkubationsmodul nach einem der vorhergehenden Ansprüche, wobei der vorbestimmte Winkelbereich einen Bereich von -35° bis +35°, vorzugsweise einen Bereich von -25° bis +25°, besonders vorzugsweise einen Bereich von - 20° bis +20° um die Nulllage herum umfasst.

9. Inkubationsmodul nach einem der vorhergehenden Ansprüche, wobei die Inkubationsrinne (2) eine in den Auslasskanal (6) mündende Öffnung entlang der Längsachse der Inkubationsrinne (2) am einen Längsende und eine in einen Einlasskanal (5) mündende Öffnung am anderen Längsende der Vertiefung (3) aufweist.

10. Inkubationsmodul nach einem der vorhergehenden Ansprüche, wobei der Träger (12) beim Bewegen bis an ein Längsende der Vertiefung (3) bewegt werden kann und dabei lückenlos an das Längsende der Vertiefung (3) anschließt.

11. Vorrichtung zur patohistologischen Untersuchung von biologischen Proben (14), umfassend eine Halterung für wenigstens ein Inkubationsmodul nach einem der Ansprüche 1 bis 10.

12. Verfahren zum Inkubieren einer vorbestimmten Anzahl von Trägern (12), umfassend die Schritte:
a) Inkubation eines Trägers (12) in einer von den Wänden der Inkubationsrinne (2) gebildeten Vertiefung (3) und einem Boden in Gegenwart einer Flüssigkeit (7), und
b) Absaugen der Flüssigkeit (7) an einem Längsende der Vertiefung (3), vorzugsweise über eine Öffnung, die in einen unter Unterdruck stehenden Auslasskanal (6) mündet,
wobei sich die Öffnung am Längsende der Vertiefung (3) befindet, vorzugsweise am Boden der Inkubationsrinne (2), und
wobei die Schritte a) und b) solange wiederholt werden bis eine vorbestimmte Anzahl von Trägern (12) in einer Vielzahl von Inkubationsrinnen (2), enthaltend in einem Inkubationsmodul (1) gemäß einem der Ansprüche 1 bis 10 oder in einer Vorrichtung gemäß Anspruch 11, mit wenigstens einer biologischen Probe (14) beladen wird.

13. Verfahren nach Anspruch 12, wobei wenigstens einer der Schritte a) und b), vorzugsweise Schritt b) unter Schwenken in einem vorbestimmten Winkelbereich durchgeführt wird.

14. Verfahren nach Anspruch 13, wobei Schritt b) in dem Moment ausgeführt wird, in dem eine Inkubationsrinne (2) derart geschwenkt wird, dass eine darin befindliche Flüssigkeit (7) sich an dem Längsende der Inkubationsrinne (2) ansammelt, an dem die Flüssigkeit (7) abgesaugt wird, und in dem vorzugsweise der Träger (12) sich bis an dieses Längsende der Vertiefung (3) bewegt hat und besonders vorzugsweise dabei lückenlos an das Längsende der Vertiefung (3) anschließt.

15. Verfahren nach einem der Ansprüche 13 bis 14, wobei ein einzelner Wipptisch (4) unabhängig von den anderen Wipptischen (4) aus dem Array (9) fixiert wird, so dass eine weitere biologische Probe auf den einzelnen Wipptisch (4) beladen wird und/oder die biologische Probe (14) von dem einzelnen Wipptisch (4) entladen wird.
